# EUROPEAN PATENT APPLICATION

(11) **EP 2 648 121 A1**
(43) Date of publication of application: **09.10.2013**
(21) Application number: 12162931.5
(22) Date of filing: 03.04.2012
(51) Int. Cl.: G06F 19/00

(54) **Analyzing an action**

(71) Applicant: Koninklijke Philips N.V., 5656 AE Eindhoven (NL)
(72) Inventor: Geleijnse, Gijs, 5600 AE Eindhoven (NL); Ivanovic, Ana, 5600 AE Eindhoven (NL); Nicolaas, Arvid Randal, 5600 AE Eindhoven (NL); Opfer, Roland Johannes, 5600 AE Eindhoven (NL)
(74) Representative: Damen, Daniel Martijn

(57) **Abstract**

A system for analyzing an action is disclosed. An action input unit (1) is arranged for enabling a user (10) to indicate a proposed action in respect of a subject. A status unit (2) is arranged for retrieving status information relating to a status of the subject from a data record. A knowledge unit (3) is arranged for retrieving a representation of knowledge from a knowledge base (12), wherein the knowledge relates possible statuses with possible actions. A matching unit (4) is arranged for matching the action and the status information with the knowledge, to obtain a representation of relevant knowledge in support of the action. An explanation output unit (5) is arranged for generating an explanation comprising at least one representation of relevant knowledge obtained by the matching unit (4).

## Description

### FIELD OF THE INVENTION

The invention relates to analyzing an action.

### BACKGROUND OF THE INVENTION

In clinical practice and more particularly in the cardiology and oncology domain, difficult decisions are taken that severely affect the patient's health and chances of survival. When decisions do not meet the patient's hopes or expectations, such decisions may be hard to convey. Also, certain decisions may be hard to accept for the patient, which hampers the immediate implementation and therefore the patient's health. Also, when no decision has been taken or it was decided not to conduct any action, proper communication may improve the patient's state-of mind while waiting for treatment.

"Building an explanation function for a hypertension decision-support system", by Ravi D. Shankar et al., in: MedInfo2001, London, UK, published in 2001, discloses a decision-support system that provides recommendations for managing hypertension in primary care. User acceptance of the system depends on how well the system explains its reasoning and justifies its conclusions. The explanation function obtains its information by tapping into EON's components, the guideline document, and medical literature. However, the system only provides explanations of the recommendations generated by the system itself.

### SUMMARY OF THE INVENTION

It would be advantageous to have an improved system for analyzing an action. To better address this concern, a first aspect of the invention provides a system, comprising
an action input unit for enabling a user to indicate a proposed action in respect of a subj ect;
a status unit for retrieving status information relating to a status of the subject from a data record;
a knowledge unit for retrieving a representation of knowledge from a knowledge base, wherein the knowledge relates possible statuses with possible actions; and
a matching unit for matching the proposed action and the status information with the knowledge, to obtain a representation of relevant knowledge in support of the proposed action.

The system thus enables a user to provide a proposed action to the system, and finds knowledge that matches the patient case and that supports the proposed action. This way, it allows the user to generate explanations more easily, even automatically. Moreover, if the user considers that the supporting knowledge is insufficient to warrant the proposed action, the user could revise its planned course of action.

The system can be used as part of a tool that assists the clinicians throughout the discharge process to optimize the hospital-to-home cycle. In this context, evidence-based tools such as the system described herein may be used to support the discharge process, tailor discharge instructions and assess the readmission risk of a hospitalized heart failure (HF) patient. The system may help with the generation of tailored discharge instructions, specific to the patient's condition, co-morbidities and context. For example, the system may be used to facilitate the communication and decision progress in tumor board meetings (or other multi-disciplinary meetings with multiple clinical experts treating a patient, such as an oncology patient).

Technology may assist in conveying clinical decisions to the patients. When automatically offering the patient personalized information, both the workflow efficiency as well as the patient's adherence may be improved.

A method to automatically generate an explanation of the clinical decision taken may be provided by the systems and methods described herein. Such an explanation may help the patient in understanding and accepting the decision. This would lead to an increase in patient satisfaction regarding the treatment in the hospital. Also, the clinical staff is helped as the tailored information is collected automatically, allowing a more efficient patient education process.

The system may comprise an explanation output unit for generating an explanation comprising at least one representation of relevant knowledge obtained by the matching unit. Such an automatically generated explanation further enhances the productivity of a user.

The system may comprise a test unit for evaluating whether the representation of relevant knowledge in support of the proposed action is sufficient to warrant the proposed action. Such test unit may assure that only appropriate actions are implemented. For example, the test unit is associated with an alert unit for alerting the user in case of an insufficiently warranted action.

The system may comprise a prioritization unit for generating a prioritization of representations of knowledge obtained by the matching unit based on a class of the knowledge represented by each representation, and wherein the explanation output unit is arranged for generating the explanation in dependence on the prioritization. This allows most important reasons to be included in an explanation with priority.

The knowledge base may comprise a rule that associates at least one precondition with at least one recommendation. The matching unit may be arranged for matching the information relating to the status of the subject and the proposed action with the precondition and the recommendation. This way, applicable rules are obtained that support the proposed action. Moreover, it allows computer readable guidelines to be matched against the current patient's case and the proposed action, to provide an explanation of the action without having to go through a process in which a decision support system generates the proposed action. Rather, the user may provide the proposed action to the system by means of an input device. For example, the decision may have been taken during a multi-disciplinary meeting of healthcare professionals. These may have taken the guidelines into account when taking their decision. The system may help to reduce the burden of documenting which rules were applied to reach the decision.

The knowledge base may comprises a plurality of guidelines, wherein each guideline comprises at least one rule that associates at least one precondition with at least one recommendation, and comprising a quality unit for determining a quality of a match of the status information and proposed action with a guideline. The quality of the match may be taken into account, to further enhance the performance in terms of accuracy of the output of the system.

The knowledge base may comprise a clinical evidence database that documents patient cases, including actions taken and effects of actions. The matching unit may be arranged for matching the status information of the subject and the proposed action against the patient cases including the actions taken. This helps to predict the effect of the action, which may be supportive of the proposed action. Consequently, this may help to justify the proposed action. Alternatively, in case of a negative effect, it may be used to counter a proposed action.

The explanation output unit may be arranged for generating an explanation comprising a predicted effect of the proposed action, based on a matching patient case. This further enhances the produced explanation.

The matching unit may be arranged for finding at least one patient case that matches the status of the subject, and wherein the proposed action was not taken. The system may be arranged in this way to predict an effect of not implementing the proposed action or of implementing an alternative action. This provides insight into the consequences of not implementing the proposed action.

The matching unit may be arranged for identifying a person involved in proposing the proposed action, and wherein the explanation unit is arranged for including information relating to authority and/or experience of the person in the explanation. This may help to increase acceptance by end users, such as patients, to accept the proposed action based on the authority and/or experience of the person who has proposed the action.

In another aspect, the invention provides a workstation comprising a system as set forth herein.

In another aspect, the invention provides a method of analyzing an action, comprising
enabling a user to indicate a proposed action in respect of a subject;
retrieving status information relating to a status of the subj ect from a data record;
retrieving a representation of knowledge from a knowledge base, wherein the knowledge relates possible statuses with possible actions; and
matching the proposed action and the status information with the knowledge, to obtain a representation of relevant knowledge in support of the proposed action.

In another aspect, the invention provides a computer program product comprising instructions arranged for causing a processing system to perform a method as set forth herein. The computer program product may also comprise an implementation of at least part of a system set forth herein.

It will be appreciated by those skilled in the art that two or more of the above-mentioned embodiments, implementations, and/or aspects of the invention may be combined in any way deemed useful.

Modifications and variations of the image acquisition apparatus, the workstation, the system, the method, and/or the computer program product, which correspond to the described modifications and variations of the system, can be carried out by a person skilled in the art on the basis of the present description.

### BRIEF DESCRIPTION OF THE DRAWINGS

These and other aspects of the invention are apparent from and will be elucidated hereinafter with reference to the drawings.
Fig. 1 illustrates a system for analyzing an action.
Fig. 2 illustrates a method of analyzing an action.
Fig. 3 illustrates an information structure.

### DETAILED DESCRIPTION OF EMBODIMENTS

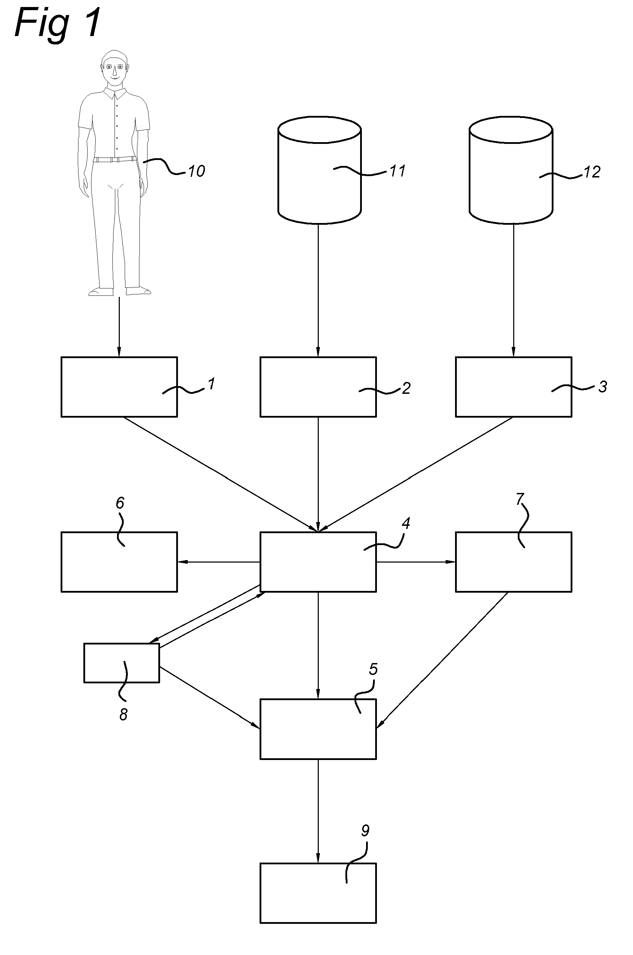

Fig. 1 illustrates a system for analyzing an action. The system may be implemented in a device using dedicated electronic circuitry. Alternatively, the system may be implemented by means of a suitably programmed computerized system. Such a system may be implemented on a distributed computer system or a standalone device. The system may comprise a workstation arranged for providing at least part of a user interface. The system may comprise a network connection port to communicate with e.g. a remotely maintained database, a user input device such as a pointer device, a keyboard, or a touch screen, and a display device.

The system may comprise an action input unit 1 arranged for receiving a representation of a proposed action in respect of a subject from a user 10. Thus, the action input unit may be arranged for enabling the user to indicate a proposed action in respect of the subject. The action input unit may be implemented by use of one or more user interface elements. The action may comprise an action to be taken in respect of a healthcare procedure of a patient for example. The indication may comprise a selection of an action from a predefined set of possible actions. Alternatively or additionally, the indication may comprise a parameter value such as a number. The indication of the action may also be provided in free-text form, by means of typing or dictation. In that case, natural language processing techniques may be employed to obtain a more structured representation of the action.

The system may comprise a status unit 2 arranged for retrieving status information relating to a status of the subject from a data record. The status unit 2 may be arranged for retrieving the status information from a cases database 11. Examples of such a cases database are a healthcare information system, a patient health record database, or a database containing other kinds of cases on any suitable types of subjects. The status information may comprise any information about the subject. For example, medical information is included in the cases database 11. Information about clinical history and of current symptoms and medical problems of the subject may be retrieved by the status unit 2.

The system may comprise a knowledge unit 3 arranged for retrieving a representation of knowledge from a knowledge base 12. That knowledge may relate possible statuses with possible actions. For example, information about a particular status may be associated in the knowledge base 12 with an action that has been proposed for that subject and/or an action that has been implemented for that patient. That status information in the knowledge base 12 may be general status information, such as a constraint on a status value in a guideline, or it may relate to a specific status of a particular subject, for example a historic patient case.

The system may comprise a matching unit 4 arranged for matching the action obtained by the action input unit 1 and the status information obtained by the status unit 2 with the knowledge obtained from the knowledge unit 3. The matching unit 4 thus extracts the knowledge that is relevant for the current case and the currently proposed action. This way, a representation of relevant knowledge in support of the action may be obtained. The matching unit 4 may comprise an implementation of any suitable kind of matching algorithm. For example, in case the knowledge contains constraints on status parameters, the algorithm may check whether the constraints are satisfied by the current case. In case the knowledge contains concrete examples of historic cases, the matching unit 4 may be arranged to find cases with a status similar to the status of the current subject (as obtained by the status unit 2).

The system may comprise an explanation output unit 5 for generating an explanation comprising at least one representation of relevant knowledge obtained by the matching unit 4. The explanation unit may, for example, output a list of suitable bits of relevant knowledge, applied to the present case. For example, the explanation output unit 5 is arranged for generating pieces of natural language text for each representation of relevant knowledge.

The system may comprise a test unit arranged 6 for evaluating whether the representation of relevant knowledge in support of the action is sufficient to warrant the implementation of the action. For example, if no relevant knowledge in support of the action matches the status information, this may cause the test unit 6 to generate a signal indicative of 'insufficient' support of the action. Alternatively, a threshold of the amount of supporting knowledge, optionally taking into account a quality factor of the knowledge, may be applied. Also, a value indicative of the amount and/or quality of the found supporting knowledge may be computed.

The system may comprise a prioritization unit 7 arranged for generating a prioritization of representations of knowledge obtained by the matching unit 4. Such a prioritization may be based on a class of the knowledge represented by each representation. Other factors that may influence the prioritization may include how well the knowledge matches the current case, and/or the reliability of the knowledge in the knowledge base 12. The explanation output unit 5 may be arranged for generating the explanation in dependence on the prioritization. For example, the knowledge is presented in the explanation in order of decreasing priority. In another example, only the knowledge with the highest priority is presented, and lower-priority is not included in the explanation.

The knowledge may comprise a rule that associates at least one precondition with at least one recommendation. This is the case, for example in a representation of a guideline. The matching unit 4 may be arranged for matching the information relating to the status of the subject and the action with the precondition and the recommendation of the rule. For example, the matching unit 4 may form a subject tuple of the status of the subject and the action, and knowledge tuples each including a precondition and an associated recommendation of a rule. The matching unit 4 may be arranged for finding the knowledge tuples that best match the subject tuple.

The knowledge may comprise a plurality of guidelines. Each guideline may comprise at least one rule that associates at least one precondition with at least one recommendation. Moreover, the system may comprise a quality unit 8 arranged for determining a quality of a match of the status and action with a guideline. Such quality of a match may be established, for example, by counting the number of constraints of the guideline that are satisfied by the status of the subject.

The knowledge base 12 may comprise a clinical evidence database that includes information about patient cases, including actions taken, and effects of actions. Such information may be used by the matching unit 4, which may be arranged for matching the status of the subject and the proposed action against the patient cases and actions taken. The effects may relate to an outcome of a treatment, quality of life, and/or expected life years, for example.

The explanation output unit 5 may be arranged for generating an explanation comprising a predicted effect of the proposed action, based on a matching patient case.

The matching unit 4 may be arranged for finding at least one reference subject case in a set of reference (or historic) subject cases that matches the status of the current subj ect, but wherein the currently proposed action was not taken. The matching unit 4 may use this information to generate a prediction of an effect of not implementing the proposed action or of implementing an alternative action. For example, if the reference subject, in which the action was not performed, had a bad treatment outcome, this could be used as suit able explanatory information.

The matching unit 4 may be arranged for identifying a person involved in proposing the proposed action. Such information may be obtained from the cases database 11 or from the user 10, or from another source. For example, information about the responsible clinician or the composition of the multi-disciplinary board, including for example amount and kind of experience and official qualifications, may be retrieved in this way. The explanation unit 5 may be arranged for including information relating to authority and/or experience of the person in the explanation.

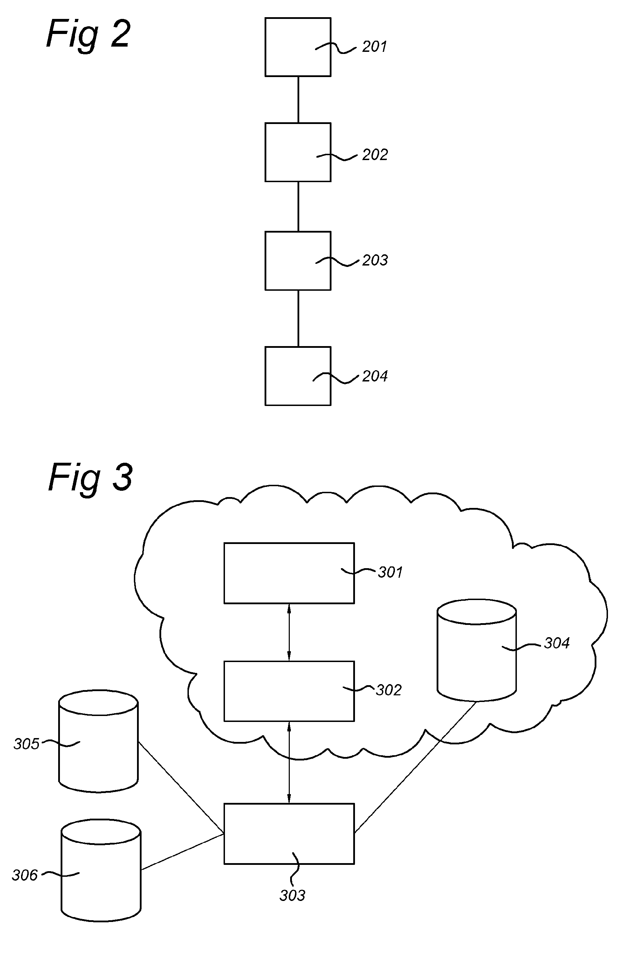

Fig. 2 illustrates a method of analyzing an action. The method may comprise step 201 of receiving a representation of a proposed action in respect of a subj ect from a user. The method may comprise step 202 of retrieving status information relating to a status of the subj ect from a data record. The method may comprise step 203 of retrieving a representation of knowledge from a knowledge base, wherein the knowledge relates possible statuses with possible actions. The method may comprise step 204 of matching the action and the status information with the knowledge, to obtain a representation of relevant knowledge in support of the action. The method may be extended and/or modified based on the functionalities described in respect of the system. The method and system may be implemented entirely or in part by means of a computer program product comprising instructions arranged for causing a processing system to perform the described functionality.

The automatic annotation of a clinical decision may be realized by automatically combining and selecting the following elements:
- *Guidelines:* An explanation that the decision meets the clinical guidelines.
- *Clinical Evidence:* Alternatively and/or additionally, an explanation that the decision is the best one based on patient population data (for the particular hospital).
- *Reversed Clinical Evidence:* Alternatively and/or additionally, an explanation of the adverse effects of not implementing the clinical decision based on patient population data.

*Authority:* Alternatively and/or additionally, an explanation why the clinical staff involved is credible to treat the patient in the optimal way.

The explanation output unit 5 may be operatively coupled to a reporting system 9. The reporting system 9 may be arranged for including the explanation obtained from the explanation output unit 5 into a report. Such a report may include further information, such as the decision itself and some information about the patient. That other information may be produced automatically or manually using a document authoring tool such as a text editor.

Fig. 3 illustrates an information structure that may be used in conjunction with the described system and/or method. The figure shows the following components.
- A board 301, such as a tumor board in a hospital, the forum which is responsible for treatment of the patient. This forum comprises at least one clinician and is typically a multi-disciplinary team of experts. An information profile of the forum or of a clinician may be included in the knowledge base 12. Such information profile may include
   - Clinical Role (medical expertise)
   - Education
   - Years of experience
   - The hospital's Performance Indicators (such as mortality, survival, readmission, patient satisfaction, success rates, infections etc.)
   - The Hospital's Performance Indicators per Disease Domain
   - The Clinician's Personal Performance Indicators
   - The Clinician's Disease-specific Performance Indicators
- EHR 304, the patient's Electronic Health Record. This record may be part of the cases database 11. This record may contain a patient's health status, co-morbidities, disease progression and earlier treatments.
- Clinical Decision 302 as formulated by the Tumor Board. The clinical decision may comprise a proposed action (a proposed action may comprise a decided action), and this action may be indicated by a user 10 of the system to the action input unit 1. This clinical decision may be documented by the recommended treatment and may comprise one or more of the following:
- The corresponding element of a predefined vocabulary with all potential treatments that be decided by the board.
   - The anticipated outcome.
   - The recommended time frame of implementation.
   - Optionally, a review of alternative treatments formulated using the predefined vocabulary and the reason for not selecting these.
- The hospital's historic patient records 305, which may be annotated with any one or more of:
   - The clinical decision
   - Its implementation
   - Outcome measured afterwards.
- A set of clinical guidelines 306 each describing a relation between a patient's condition as expressed in an EHR and a treatment guideline. The guidelines may be formulated as follows. Their input may comprise a combination of predicates over elements of the EHR (e.g. a range for systolic blood pressure, presence or absence of co-morbidities and non-response to a certain treatment or medication dosage). The outcome of these guidelines may comprise a clinical decision that is restricted to the vocabulary as described above. If guidelines are gathered from multiple sources/authorities, the most specific one may be selected using the level of evidence on which the guideline is based and the class of the recommended guideline. Using such prioritization, the most specific one or ones may be identified and used in the report. These guidelines may be annotated with background information such as its source, date and the reason for its formulation.

These elements may be combined by a patient report algorithm into a text describing the clinical decision and its argumentation. The argumentation may comprise any one or more of the following elements.
*1. Guidelines:* If the EHR-Clinical Decision combination meets any of the guidelines in the Clinical Guidelines Database, a text may be selected that explains the guideline and its background.
*2. Clinical Evidence:* If the clinical decision is the one most frequently taken for patients in similar conditions, or historic evidence suggests that its expected outcome is a beneficial one, a text may be generated containing this evidence.
*3. Reversed Clinical Evidence:* Historic patient cases with similar EHR conditions may be collected. The outcomes for alternative clinical decisions may be evaluated and presented in a text.
*4. Adverse Effects:* Additionally, the outcomes for not implementing the chosen clinical decision, based on comparable cases, may be extracted from the knowledge database 12 and presented in a text.
*5. Authority:* A textual explanation on the credibility and experience of the clinical staff involved in the tumor board may be extracted and presented.

These elements may be constructed and combined using the following method. However, this is not a limitation. Alternative methods will be apparent to the person skilled in the art based on the present disclosure.
1. The tool administrator may be invited to specify the priority queue of the argument classes as listed above. The most convincing argument classes or the classes with the highest persuasive influence are given the highest priority. Also, the administrator may indicate the maximum number of arguments to be included in the information letter.
2. [Guideline check] The clinical decision D may be retrieved and queried in the clinical guidelines database. All guidelines that lead to D may be analyzed. These guidelines may be compared with the EHR of the patient. If exactly one guideline matches the patient's EHR, then a predefined text may be retrieved that is associated with the guideline. This text template may be personalized by entering elements of the EHR into the text at predefined placeholders.

If multiple guidelines match the patient's EHR, then these guidelines may be ranked according to the quality score of the guidelines. Elements that contribute to a high quality score of any particular subject may include specificity, novelty of the guideline, reputation of the institute formulating the guideline and evidence of the quality of the guideline. These elements may be combined into the quality score that is associated with each guideline. The matching guideline with the highest quality score may be then selected.
3. [Clinical Evidence] Using a similarity matching algorithm, historic patient records may be retrieved that strongly resemble the current patient case. For these patient cases, all treatments may be observed and their effects may be extracted from the patient record at a later moment in time. In this way, statistics may be collected about the effects of the alternative treatments of similar patients. If these statistics are favorable for the patient case, a text may be composed describing the effectiveness of the treatment using the generated numbers. Additionally, for all treatments found that were offered to the resembling patients, the overall effectiveness may be computed. This may be done by querying the database for all patients receiving these particular treatments and observing their health statuses in a later moment in time. When these statistics are favorable for the clinical decision taken by the board, a text fragment describing these statistics may be added to the clinical evidence text fragment.
4. [Reversed Clinical Evidence] The historic patient records may be collected for which decision D has been taken, but has not been implemented. For these patients, the health outcomes may be observed in the period after the decision was taken. Additionally, the combined health outcomes for all alternative treatments may be collected. These two figures may be compared with the derived outcomes for the treatment corresponding to D. If the implementation of D provides the patient with better perspectives, then these figures may be included into a predefined text fragment. If D does not provide better perspectives, another kind of predefined operation may be performed, for example issuing an alert to the user or skipping the argument.
5. [Adverse Effects] The outcomes for patients with recommendation D, which has not been followed up may be analyzed. The adverse effects of not following up the decision D by this patient population (mortality, readmission, infections, complications etc.) may be included into a predefined text fragment template.
6. [Authority] Texts describing the background of the (main) members of the tumor board may be selected from a database. These texts may be personalized to the clinician by extracting their personalized data form a database, such as years of experience. Additionally, biographic information may be included in the form of free text fields, which have been collected in the database. Apart from the general biographical information of the tumor board members (including photographs), their expertise specific to the patient case and the clinical decision may be taken. This information may collect the years of experience and the number of patients treated a.) with similar conditions, b.) with the decided treatment and c) the success rate.
7. Having selected a prioritized list of text fragments, these fragments may be combined into a report. Alternatively, the user of the tool (i.e. the clinician responsible for informing the patient) may review all generated text fragments and select the ones to be included into the patient report.
8. Optionally, using the scheduling service of the hospital, the information about the next appointment with one of the members of the tumor board may be included in the report. For example, a sentence similar to the following may be included. "Dr. Bakker (image) will provide this treatment on June 25th at 14.30. His assistant, Mrs. Slager will be expecting you at 11.00 at department TH1".

It will be appreciated that the invention also applies to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise calls to each other. An embodiment relating to a computer program product comprises computer-executable instructions corresponding to each processing step of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a storage medium, such as a ROM, for example, a CD ROM or a semiconductor ROM, or a magnetic recording medium, for example, a flash drive or a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system for analyzing an action, comprising
an action input unit (1) for enabling a user (10) to indicate a proposed action in respect of a subj ect;
a status unit (2) for retrieving status information relating to a status of the subject from a data record;
a knowledge unit (3) for retrieving a representation of knowledge from a knowledge base (12), wherein the knowledge relates possible statuses with possible actions; and
a matching unit (4) for matching the proposed action and the status information with the knowledge, to obtain a representation of relevant knowledge in support of the proposed action.

2. The system according to claim 1, comprising an explanation output unit (5) for generating an explanation comprising at least one representation of relevant knowledge obtained by the matching unit (4).

3. The system according to claim 1, comprising a test unit (6) for evaluating whether the representation of relevant knowledge in support of the proposed action is sufficient to warrant the proposed action.

4. The system according to claim 2, further comprising a prioritization unit (7) for generating a prioritization of representations of knowledge obtained by the matching unit (4) based on a class of the knowledge represented by each representation, and wherein the explanation output unit (5) is arranged for generating the explanation in dependence on the prioritization.

5. The system according to claim 1, wherein the knowledge base (12) comprises a rule that associates at least one precondition with at least one recommendation, and the matching unit (4) is arranged for matching the information relating to the status of the subject and the proposed action with the precondition and the recommendation of the rule.

6. The system according to claim 5, wherein the knowledge base (12) comprises a plurality of guidelines, wherein each guideline comprises at least one rule that associates at least one precondition with at least one recommendation, and comprising a quality unit (8) for determining a quality of a match of the status information and the proposed action with a guideline.

7. The system according to claim 1, wherein the knowledge base (12) comprises a clinical evidence database that documents patient cases, actions taken, and effects of actions, and wherein the matching unit (4) is arranged for matching the status information of the subject and the proposed action against the patient cases and actions taken.

8. The system according to claim 7, comprising the explanation output unit (5) for generating an explanation comprising a predicted effect of the proposed action, based on a matching patient case.

9. The system according to claim 7, wherein the matching unit (4) is arranged for finding at least one patient case that matches the status of the subject, and wherein the proposed action was not taken, to predict an effect of not implementing the proposed action or of implementing an alternative action.

10. The system according to claim 2, wherein the matching unit (4) is arranged for identifying a person involved in proposing the proposed action, and wherein the explanation unit (5) is arranged for including information relating to authority and/or experience of the person in the explanation.

11. A workstation comprising a system according to claim 1.

12. A method of analyzing an action, comprising
enabling (201) a user to indicate a proposed action in respect of a subject;
retrieving (202) status information relating to a status of the subject from a data record;
retrieving (203) a representation of knowledge from a knowledge base, wherein the knowledge relates possible statuses with possible actions; and
matching (204) the proposed action and the status information with the knowledge, to obtain a representation of relevant knowledge in support of the proposed action.

13. A computer program product comprising instructions arranged for causing a processing system to perform the method according to claim 12.
